# EUROPEAN PATENT SPECIFICATION

(11) **EP 4 065 336 B1**
(45) Date of publication and mention of the grant of the patent: **26.07.2023**
(21) Application number: 20811366.2
(22) Date of filing: 27.11.2020
(51) Int. Cl.: B29C 48/92, B29C 48/25, B29B 7/72, B29B 9/06, B29B 7/74, B29B 9/16, B29B 7/42, B29B 7/46

(54) **METHOD AND CONTROLLING SYSTEM FOR CONTROLLING POLYMER VISCOSITY QUALITY**
VERFAHREN UND STEUERUNGSSYSTEM ZUR STEUERUNG DER POLYMERVISKOSITÄTSQUALITÄT
PROCÉDÉ ET SYSTÈME DE CONTRÔLE DE LA QUALITÉ DE VISCOSITÉ DE POLYMÈRES

(30) Priority: 28.11.2019 EP 19212098
(43) Date of publication of application: 05.10.2022
(73) Proprietor: BASF SE, 67056 Ludwigshafen am Rhein (DE)
(72) Inventor: OEHME, Felix, 01097 Dresden (DE); SOCHER, Robert, 01986 Schwarzheide (DE); KOYCHEVA, Evelina, 01986 Schwarzheide (DE)
(74) Representative: BASF IP Association
(86) International application number: PCT/EP2020/083580
(87) International publication number: WO 2021/105325

(56) References cited:
- EP-A1- 3 020 530
- EP-A1- 3 220 126
- WO-A2-2008/040943
- ES-A1- 2 331 720
- KUMAR A ET AL: "A model based approach for estimation and control for polymer compounding (april 2003)", PROCEEDINGS OF THE 2003 IEEE INTERNATIONAL CONFERENCE ON CONTROL APPLICATIONS. CCA 2003. ISTANBUL, TURKEY, JUNE 23 - 25, 2003; [IEEE INTERNATIONAL CONFERENCE ON CONTROL APPLICATIONS], NEW YORK, NY : IEEE, US, vol. 1, 23 June 2003 (2003-06-23), pages 729-735, XP010652701, ISBN: 978-0-7803-7729-5
- XUEQIN LIU ET AL: "'Soft-sensor' for real-time monitoring of melt viscosity in polymer extrusion process", DECISION AND CONTROL (CDC), 2010 49TH IEEE CONFERENCE ON, IEEE, 15 December 2010 (2010-12-15), pages 3469-3474, XP031915619, DOI: 10.1109/CDC.2010.5717800 ISBN: 978-1-4244-7745-6 cited in the application

## Description

### Technical Field

The invention relates to a method for controlling polymer viscosity quality in a compounding process as well as to a computer program and a controlling system for controlling polymer viscosity quality in a compounding process. Such methods, computer programs and systems can, in general, be employed for polymer processing in chemical industry e.g. for synthesis and modification of polymer products. However, further applications are possible.

### Background art

Compounding processes, such as plastic compounding processes, are common manufacturing processes in small and large scale industry. Generally, in compounding processes, polymers and additives are blended, usually in an extrusion process. In typical compounding processes a polymer and one or more additives are melted, usually in a heating process, and then mixed and pushed through a die, e.g. under an applied pressure, creating a plastic strand. The plastic strand is then hardened, usually in a cooling process, and granulated, such as by using a granulator breaking the plastic strand into granulate pellets.

Polymer materials and additives are usually heat and/or pressure sensitive materials carefully balanced and selected in order to obtain desired properties and high quality standards. In particular, a melt viscosity is a relevant indicator of melt quality in the process of compounding. Specifically, the melt viscosity may indicate mechanical and aesthetic properties and may be an important quantity for any processing chain of plastic materials. Thus, generally, after start of a production line, a sample of the melt is taken when the process is stable and the extruder is stopped to wait for a laboratory result of the sample. Thus, in order to minimize production rejects and waste, as well as for conserving resources, compounding processes, specifically compounding process parameters, such as heat, pressure and viscosity of the molten mass of material, are typically monitored carefully. However, in-line rheometer, e.g. for monitoring melt viscosity, usually show a measurement delay and/or interfere with the compounding process such as by disturbing a flow of the molten mass of material. As an example, in "A novel approach to dynamic modelling of polymer extrusion for improved process control" on pages 617 - 628 in "proceedings of the Institution of Mechanical Engineers, Vol. 221 Part I: J. Systems and Control Engineering", McAfee and Thompson describe an approach to the problem by the modelling of extrusion viscosity and pressure, adopting a grey box modelling technique that combines mechanistic knowledge with empirical data using a genetic algorithm approach.

Further, in "Soft-sensor for real-time monitoring of melt viscosity in polymer extrusion process" in "49th IEEE Conference on decision and Control", Liu et al. propose a soft-sensor involving a non-linear finite impulse response model with adaptable linear parameters for real-time prediction of the melt viscosity based on the process inputs. The model output is then used as an input of a model with a simple fixed structure to predict the barrel pressure, which can be measured online. Finally, the predicted pressure is compared to the measured value and the corresponding error is used as a feedback signal to correct the viscosity estimate.

Still, when controlling polymer viscosity quality in compounding processes, several technical challenges remain. Typically, monitoring and/or simulating polymer viscosity in a compounding process require extensive modelling and the performance of complex calculations and intensive computing. The methods and systems typically require large data storage and computing capacities as well as technical expertise, which often are not available. Thus, generally, the performing of such methods is very time-consuming and complex.

WO 2008/040943 A2 describes a method of determining the viscosity of an extrudate material in an extrusion system, comprising the steps of (i) acquiring measurements of one or more process conditions of the extrusion system, (ii) acquiring indications of throughput of the extrudate material through the extrusion system, (iii) acquiring pressure measurements of the extrudate material at one or more locations of the extrusion system, (iv) using a first model to generate an estimated viscosity of the extrudate material using data relating to the material and one of the measurements or each process condition, (v) using a second model to generate an estimated pressure of the extrudate material using data relating to the extrusion system geometry, the estimated viscosity of the extrudate material and one of the indications of the extrudate material throughput, (vi) comparing the estimated pressure of the extrudate material with a pressure measurement of the extrudate material to generate a pressure difference value, (vii) using the pressure difference value to generate a viscosity correction value, (viii) feeding the viscosity correction value into the first model and using the viscosity correction value in the generation of a next estimated viscosity of the extrudate material, and (ix) repeating steps (iii) to (viii) one or more times, each repetition using another of the measurements of the or each process condition and another of the indications of the extrudate material throughput, wherein the generated pressure difference values are driven towards a minimum value.

ES 2 331 720 A1 describes a method which includes at least the steps of studying values of manufacturing process variables, comparing said values with previously defined quality values, obtaining a set of models using techniques based on artificial intelligence, thereby obtaining a prediction of real-time quality value estimates from the values of the manufacturing process variables at every instant, and identifying lengths of the sections that fulfil or fall short of established quality criteria.

EP 3 020 530 A1 describes a simulation apparatus which includes an 1D analysis unit which performs a low-dimensional fluid-flow analysis of material in an arithmetic object field of a kneading device, based on setting information including physical property of the material, and configuration data and an operation condition of the kneading device for kneading the material, an area selection unit which receives selection of an object area as an object of a high-dimensional fluid-flow analysis in the arithmetic object field, and a 3D analysis unit which extracts physical quantities of the material relating to the object area, based on a result of the low-dimensional fluid-flow analysis, and performs a high-dimensional fluid-flow analysis of the material in the object area, based on the extracted physical quantities and the setting information.

Kumar A. et al.: "A model based approach for estimation and control for polymer compounding", Proceedings of the 2003 IEEE International Conference on Control Applications, CCA 2003, ISTANBUL, TURKEY, JUNE 23 - 25, 2003; NEW YORK, NY: IEEE, US, vol. I, 23 June 2003, pages 729-735, XP010652701, ISBN: 978-0-7803-7729-5, describes a framework for operation of extruders incorporating intelligent means for (i) online product quality estimation (inferential sensing), (ii) diagnostics for common process/material failures, and (iii) closed-loop control of product quality based on the on-line estimation. A model-based approach is used for the estimation, diagnostics and controls in a unified framework.

### Problem to be solved

It is therefore desirable to provide means and methods, which address the above mentioned technical challenges of controlling polymer viscosity quality in a compounding process of polymers using at least one extruder. Specifically, methods, computer programs and systems shall be proposed for improving process of controlling polymer viscosity quality in a compounding process of polymers using at least one extruder, compared to methods and systems known in the art.

### Summary

This problem is addressed by the methods, computer programs and systems of the independent claims. Advantageous embodiments, which might be realized in an isolated fashion or in any arbitrary combinations, are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention a method according to claim 1 for controlling polymer viscosity quality in a compounding process of polymers using at least one extruder is proposed. The method steps may be performed in the given order. Further, one or more than one or even all of the steps may be performed once or repeatedly. Further, one or more than one or even all of the steps may be performed once or repeatedly. Further, the method steps may be performed in a timely overlapping fashion or even in parallel.

The term "polymer" as used herein is a broad term and is to be given its ordinary and customary meaning to a person of ordinary skill in the art and is not to be limited to a special or customized meaning. The term specifically may refer, without limitation, to a substance composed of a plurality of macromolecules, such as of a covalently bonded chain or network of molecules. Specifically, in a compounding process, the polymer may be combined with further substances such as fillers and/or additives to form a compound. However, the polymer, for example, may comprise fillers and/or additives even before being combined with at least one further substance in the compounding process. As an example, the polymer may be or may comprise a precursor and/or a preliminary stage of the compound.

In particular, the polymer may comprise one or more characteristics, such as for example a polymer viscosity. The term "viscosity" as used herein is a broad term and is to be given its ordinary and customary meaning to a person of ordinary skill in the art and is not to be limited to a special or customized meaning. The term specifically may refer, without limitation, to a resistance to deformation, specifically in reaction of an arbitrary material to an external force applied onto the material. The term "polymer viscosity" as used herein is a broad term and is to be given its ordinary and customary meaning to a person of ordinary skill in the art and is not to be limited to a special or customized meaning. The term specifically may refer, without limitation, to a resistance to deformation of a polymer. Specifically, the polymer viscosity may be or may comprise a rheological property of the polymer, such as of a substance composed of a plurality of macromolecules. In particular, the polymer viscosity may be dependent on at least one external influence, e.g. temperature and/or pressure, affecting the polymer. The polymer viscosity may be or may comprise a material property of the polymer evolving over one or more of time, temperature and pressure. In particular, the polymer viscosity may be or may comprise an indication of a state of the covalent bonds of the molecules of the polymer. Thus, the polymer viscosity may be or may comprise information on an ability of the polymer to be mixed and/or blended with an arbitrary material, a degree of polymerization or the like. In particular, in the method for controlling polymer viscosity quality in a compounding process of polymers using at least one extruder, the term "viscosity" may specifically refer to a viscosity of the polymer after compounding. Specifically, a viscosity number may be or may comprise a measurement value describing viscosity of a pure polymer. In order to determine the viscosity number, in a first analysis step, all additives and/or additional elements of the compound may be extracted from the polymer in a laboratory. The method may specifically be suited for determining and/or predicting the viscosity of the polymer after compounding.

Specifically, the polymer viscosity may affect a polymer viscosity quality. The term "polymer viscosity quality" as used herein is a broad term and is to be given its ordinary and customary meaning to a person of ordinary skill in the art and is not to be limited to a special or customized meaning. The term specifically may refer, without limitation, to a polymer's value and/or worth dependent on the polymer's current viscosity and/or history of viscosity. In particular, the polymer viscosity quality may be or may comprise an indication on a property and/or characteristic of the polymer, e.g. in a prospective view.

The term "influence variable" as used herein is a broad term and is to be given its ordinary and customary meaning to a person of ordinary skill in the art and is not to be limited to a special or customized meaning. The term specifically may refer, without limitation, to a parameter suitable and/or configured for manipulation purposes and/or having an impact on arbitrary parameters of the process. Thus, the term "influencing variable affecting viscosity of the compound" may generally refer to a parameter suitable and/or configured for manipulating the viscosity, such as a resistance to deformation, of the compound.

The term "determine" as used herein is a broad term and is to be given its ordinary and customary meaning to a person of ordinary skill in the art and is not to be limited to a special or customized meaning. The term specifically may refer, without limitation, to a process of retrieving information. In particular, the term determine may comprise, but not be limited to, one or more of measuring, observing, reading, predicting and gathering information. In particular, with respect to the "influence variable", the term "determining the influence variable" may refer to a measuring by using the at least one sensor.

The term "sensor" as used herein is a broad term and is to be given its ordinary and customary meaning to a person of ordinary skill in the art and is not to be limited to a special or customized meaning. The term specifically may refer, without limitation, to an arbitrary element configured for detecting at least one measurable variable and generating at least one corresponding signal. Specifically, the sensor may be suitable for generating one or more of an electrical signal, an analogous signal and a digital signal, corresponding to the influence variable.

The term "viscosity of the compound" as used herein is a broad term and is to be given its ordinary and customary meaning to a person of ordinary skill in the art and is not to be limited to a special or customized meaning. The term specifically may refer, without limitation, to a resistance to deformation of the compound comprising the at least one polymer. The term "expected viscosity" as used herein is a broad term and is to be given its ordinary and customary meaning to a person of ordinary skill in the art and is not to be limited to a special or customized meaning. The term specifically may refer, without limitation, to a predicted viscosity value. Therein, the term "predicting" specifically may refer, without limitation, to determining a viscosity value for a compound material for at least one stage in the compounding process, in particular regarding time such as a time point in the future, and/or regarding a spatial position such as a different location, for example reached by the compound at a later stage in the compounding process. As an example, the expected viscosity may be or may comprise a viscosity forecast. In particular, the expected viscosity of the compound may be determined by using at least one prediction unit. In particular, with respect to the "expected viscosity", the term "determining the expected viscosity" may refer to a predicting the at least one expected value of the viscosity.

The term "prediction unit" as used herein is a broad term and is to be given its ordinary and customary meaning to a person of ordinary skill in the art and is not to be limited to a special or customized meaning. The term specifically may refer, without limitation, to an arbitrary element or system configured for determining at least one expected value of at least one target variable for at least one input variable, such as a measured and/or pre-determined input variable. The target variable may be the expected viscosity. The input variable may be or may comprise the at least one influence variable affecting viscosity of the compound. Specifically, the prediction unit may be configured for foretelling information, such as an expected and/or predicted characteristic and/or property of an arbitrary material, e.g. the expected viscosity of the compound. As an example, the prediction unit may be configured for predicting the expected viscosity of the compound by considering the at least one influence variable, such as the influence variable affecting the viscosity of the compound. The prediction unit may comprise at least one processing unit such as a processor, microprocessor, or computer system configured for predicting the at least one expected value, in particular for executing a program, specifically a software and/or application, and/or a logic in a given algorithm. The prediction unit may be configured for performing and/or executing at least one trained model of an analysis tool.

In particular, the prediction unit comprises at least one analysis tool. The term "analysis tool" as used herein is a broad term and is to be given its ordinary and customary meaning to a person of ordinary skill in the art and is not to be limited to a special or customized meaning. The term specifically may refer, without limitation, to at least one tool configured for providing and/or comprising computer readable instructions to the prediction unit for determining the expected viscosity. As an example, the analysis tool may be or may comprise at least one program, specifically a software and/or application, and/or a logic in a given algorithm. In particular, the analysis tool may be configured for examining and/or analyzing sensor data, such as data and/or information determined by using the sensor. Specifically, the analysis tool may be configured for examining and/or analyzing the at least one influence variable determined by using the at least one sensor.

The analysis tool comprises at least one trained model. The term "trained model" as used herein is a broad term and is to be given its ordinary and customary meaning to a person of ordinary skill in the art and is not to be limited to a special or customized meaning. The term specifically may refer, without limitation, to at least one mathematical model configured for predicting the target variable for the input variable. In particular, the trained model may be or may comprise an algorithm and/or neutral network trained for simulating an evolution of the viscosity. The trained model may be trained on training data. The term "training data" as used herein is a broad term and is to be given its ordinary and customary meaning to a person of ordinary skill in the art and is not to be limited to a special or customized meaning. The term specifically may refer, without limitation, to a data used for training the trained model. The training data may comprise a plurality of training data sets. Specifically, the training data may be or may comprise historical experimental data such as previously determined data, e.g. previously determined influence variables affecting viscosity.

As an example, the trained model may be configured for simulating a behavior and/or an evolution of the viscosity, thereby determining the expected viscosity. Specifically, the expected viscosity may be determined based on at least one influence variable. In particular, the trained model may be configured for simulating a course of polymer viscosity, e.g. for determining an expected viscosity, during the compounding process within an extruder. The prediction unit may be configured for computer simulation and/or for simulating a behavior and/or evolution, such as a progress in time, of a specific type of information and/or data.

The term "pre-defined threshold value" or "pre-determined threshold value" as used herein is a broad term and is to be given its ordinary and customary meaning to a person of ordinary skill in the art and is not to be limited to a special or customized meaning. The term specifically may refer, without limitation, to at least one arbitrary pre-known numerical value, which quantifies at least one limit and/or at least one boundary and/or a range. As an example, the pre-defined and/or pre-determined threshold value may be or may comprise a numerical value defining a limit and/or boundary of the compound viscosity above which the polymer generally may start to degrade. With regard to degradation processes and mechanisms reference, for example, may be made to Beständigkeit von Kunststoffen by Gottfried W. Ehrenstein and Sonja Pongratz Munich: Hanser of 2007.

The term "item of output information" as used herein is a broad term and is to be given its ordinary and customary meaning to a person of ordinary skill in the art and is not to be limited to a special or customized meaning. The term specifically may refer, without limitation, to an indication or information regarding the outcome of a comparison. In particular, the item of output information may be or may comprise indication of information on the comparison between the expected viscosity of the compound and the pre-defined and/or pre-determined threshold value. Thus, as an example, the item of output information may be generated depending on the comparison between the expected viscosity of the compound and the pre-known threshold value, such that the item of output information indicates information on the outcome of the comparison. Specifically the item of output information may comprise handling recommendation for at least one setting of the extruder.

The term "handling recommendation" as used herein is a broad term and is to be given its ordinary and customary meaning to a person of ordinary skill in the art and is not to be limited to a special or customized meaning. The term specifically may refer, without limitation, to an information comprising at least one instruction and/or at least one advice on an action and/or a practice. As an example, the handling recommendation may be or may comprise at least one instruction regarding at least one setting of the extruder. Specifically, the handling recommendation may be or may comprise information on an action to be performed with regard to the extruder, specifically on an adaption of at least one setting of the extruder.

The term "extruder" as used herein is a broad term and is to be given its ordinary and customary meaning to a person of ordinary skill in the art and is not to be limited to a special or customized meaning. The term specifically may refer, without limitation, to a system configured for at least partially plasticizing a raw material and pushing the plasticized material through a die. In particular, the extruder may be configured for generating an extrudate having an essentially constant cross-section, such as a fixed cross-section. As an example, the raw material may be or may comprise the polymer, e.g. a polymer material. Specifically, the extruder may be configured for plasticizing the polymer material, e.g. by melting the polymer, usually in a heating process, and subsequently pushing the polymer through a die, such as by applying pressure. As an example, the extruder may be selected from the group consisting of: a screw extruder, a twin-screw extruder, a planetary roller extruder. In a preferred embodiment, the extruder may be at least one twin-screw extruder. Thus, as an example, the extruder may comprise at least two screws, such as two co-rotating screws, at least one housing enclosing the screws and at least one die and/or form.

The term "setting of the extruder" as used herein is a broad term and is to be given its ordinary and customary meaning to a person of ordinary skill in the art and is not to be limited to a special or customized meaning. The term specifically may refer, without limitation, to a configuration of an extruder described by at least one parameter. In particular, the setting of the extruder may refer to a configuration of the extruder suitable for being changed and/or adapted when the extruder is running, such as during a running extrusion. Specifically, an adaption of the setting of the extruder may lead to an adaption of the process parameter, specifically of a compounding process parameter.

The term "display" as used herein is a broad term and is to be given its ordinary and customary meaning to a person of ordinary skill in the art and is not to be limited to a special or customized meaning. The term specifically may refer, without limitation, to an illustrating user interface configured for representing information in a visual form. In particular, the term "display device" may refer, without limitation, to an arbitrary element comprising the at least one display, such as at least one screen. For example the screen may have a flat and/or even surface. As an example, the display device may be or may comprise a liquid-crystal display (LCD), such as a flat-panel display, e.g. an electronically modulated optical device, using light-modulating properties of liquid crystals. Other types of displays may be possible, such as light-emitting diode (LEC) displays or the like. The display device may be part of a controlling system for controlling the extruder.

The compound may at least partially comprise at least one thermoplastic material selected from the group consisting of: polybutylene terephthalate (PBT); polyethylene terephthalate (PET); polyamide (PA); polystyrene (PS).

The influence variable may specifically be selected from the group consisting of: a head pressure; a torque, specifically a torque of an extrusion screw; a temperature, specifically a temperature of a head or flow front of a compound, e.g. a temperature of a head or flow front of a compound melt, a temperature of an extrusion screw, a temperature of a compound melt at an extrusion head or a temperature of a housing; a transfer rate; a rotational speed; a shear rate; a mass flow, specifically a mass flow rate of a polymer and/or of a compound; an electric power consumption, specifically an electric power consumption of an extruder.

Specifically, the influence variable may comprise a head pressure and/or a temperature of a housing and/or a temperature of a compound melt at an extrusion head and/or a torque, specifically a torque of an extrusion screw, and/or an electric power consumption, specifically an electric power consumption of an extruder, and/or a rotational speed and/or a mass flow.

The method may further comprise generating at least one database comprising one or both of quantitative and qualitative information on the compounding process.

The term "database" as used herein is a broad term and is to be given its ordinary and customary meaning to a person of ordinary skill in the art and is not to be limited to a special or customized meaning. The term specifically may refer, without limitation, to an arbitrary collection and/or accumulation of information. Specifically, the database may be or may comprise an accumulation of information that may be sorted and/or linked such as to facilitate a search of information within the database. Therein the term "sorted" may generally refer to a referencing and/or cross-referencing of information. The database may comprise at least one lookup table and/or at least one spreadsheet and/or at least one electronically managed chart.

In particular, the information stored in the database may specifically be selected from the group consisting of: sensor data, specifically sensor data retrieved in step a) and a respective time stamp; a material, specifically information on a material composition, in particular a reactant supplier and a specification of one or more additives; a process parameter of the compounding process, specifically an extruding parameter, such as a maximum or minimum transfer rate, or a technical design information, such as single extrusion screw or twin extrusion screws, a pre-determined target viscosity value. The database may comprise information gathered from a plurality of different databases, specifically from a plurality of differently structured databases.

The trained model may be or may comprise at least one random forest algorithm. Additionally or alternatively, further models are possible, such as at least one neural network such as at least one Multi-Layer Perceptron (MLP) neural network; at least one linear model; at least one non-linear model; at least one grey box model; and/or at least one elastic net algorithm. Specifically, the trained model may be or may comprise at least one model selected from the group consisting of: at least one random forest algorithm; at least one neural network such as at least one Multi-Layer Perceptron (MLP) neural network; at least one linear model; at least one non-linear model; at least one grey box model; at least one elastic net algorithm.

The term "grey box model" as used herein is a broad term and is to be given its ordinary and customary meaning to a person of ordinary skill in the art and is not to be limited to a special or customized meaning. The term specifically may refer, without limitation, to a model configured for combining a partial theoretical structure with data, e.g. for completing the model. In particular, the grey box model may refer to a model that comprises first-principles parts, so called white box, as well as data-driven parts, so called black box, see e.g. review paper of Von Stoch et al., 2014, Computers & Chemical Engineering, 60, Pages 86 to 101.

The term "random forest algorithm" as used herein is a broad term and is to be given its ordinary and customary meaning to a person of ordinary skill in the art and is not to be limited to a special or customized meaning. The term specifically may refer, without limitation, to an ensemble learning method configured for at least classification and/or regression. Specifically, the random forest algorithm may be configured for constructing one or more decision trees, e.g. at a training time, and outputting at least one class selected from the mode of the classes and the mean prediction of the individual trees. In particular, the random forest algorithm may show an increased flexibility compared to linear models and may thus comprise the ability to map nonlinear effects. Specifically, for the random forest algorithm knowledge on interactions between influencing variables may not be required. The random forest algorithm may allow significance analyses of influencing variables. Further, when using the random forest algorithm no danger of overfitting may exist. In particular, the random forest algorithm may be particularly suitable for classification tasks, for example, due to its structure based on at least one decision tree. Thus, as an example, the random forest algorithm may allow for a classification of a plurality of products within one model, such as within one regression model. The random forest algorithm may generally be known, e.g. from journal paper by Leo Breiman, "Random Forests" in Machine Learning 45.1, Oct. 2001, from "The Elements of Statistical Learning" by Trevor Hastie, Robert Tibshirani and Jerome Friedman, Springer New York, 2009 and from methodology article "Conditional variable importance for random forests" by Carolin Strobl et al. in BMC Bioinformatics 9.1, July 2008, page 307.

Specifically, using the random forest algorithm in the present case may allow for a reduced computing effort compared to other algorithms and models. Specifically, when compared to a genetic algorithm, in the present application, the random forest algorithm may significantly reduce computing effort. Thus, when compared to other algorithms and models, as an example, the random forest algorithm may need less computing time and therefore may be more efficient. Further, the random forest algorithm may specifically have more robust features and properties. Thus, as an example, the random forest algorithm may be less prone to interferences, such as interferences, for example, triggered by faulty data and/or measurement noise.

The term "Multi-Layer Perceptron neural network" or "MLP neural network" as used herein is a broad term and is to be given its ordinary and customary meaning to a person of ordinary skill in the art and is not to be limited to a special or customized meaning. The term specifically may refer, without limitation, to a class of feedforward artificial neural networks.

The method may further comprise automatically retrieving information from the database and providing the information to the prediction unit. In particular, the retrieving of information from the database may be performed between step a) and step b).

The term "automatically retrieving information" as used herein is a broad term and is to be given its ordinary and customary meaning to a person of ordinary skill in the art and is not to be limited to a special or customized meaning. The term specifically may refer, without limitation, to a process of gathering data autonomously performed by a computer. Thus, the information from the database may be retrieved independently, specifically without interference of a user and/or operator of the compounding process.

Step a) may further comprise at least one pre-processing step. In the pre-processing step, as an example, data determined by the sensor may be pre-processed. In particular, the pre-processing may comprise eliminating of outliers. Specifically, outliers may be eliminated from the data determined by the sensor by using at least one clustering algorithm, specifically one or more of an expectation-maximization algorithm, a k-means algorithm and a k-median algorithm.

The term "pre-processing step" as used herein is a broad term and is to be given its ordinary and customary meaning to a person of ordinary skill in the art and is not to be limited to a special or customized meaning. The term specifically may refer, without limitation, to an arbitrary act and/or step of preparation and/or treatment preceding a performance of a main step. In particular, the pre-processing may be or may comprise an initial step, such as an initial preparation and/or treatment. Thus, the term "pre-processing of data determined by the sensor" may be or may comprise an initial preparation of sensor data.

Further, in the method, a distinction is made with regard to the handling information comprised by the item of output information. Thus:
i) if the at least one expected viscosity of the compound falls within a predefined tolerance range of the threshold value, the handling recommendation comprises information on maintaining the setting of the extruder; and
ii) if the at least one expected viscosity of the compound differs from the threshold value by more than the predefined tolerance range, the handling recommendation comprises information on changing the setting of the extruder such as information on changing a rotational speed of the extruder.

The predefined tolerance range is one or both of a relative and an absolute tolerance. The predefined tolerance range is ± 5 %, preferably ± 2 %. Additionally or alternatively, the predefined tolerance range is ± 3 ml/g, preferably ± 2 ml/g.

Step c) may further comprise comparing the expected viscosity of the compound with at least one target specification, wherein the target specification may specifically be a customer specification, such as a specification given and/or set by a customer and/or buyer of the compound.

The output information may further comprise at least one information selected from the group consisting of: an information on the expected viscosity of the compound; an information on historical viscosity of the compound such as measured viscosity and/or expected viscosity.

As an example, the method may further comprise regulating at least one process parameter of the compounding process depending on the output information. In particular, the process parameter to be regulated may specifically be or may comprise a rotational speed of the extruder. Thus, as an example, when increasing the rotational speed, the viscosity, such as the polymer viscosity, may be reduced and vice versa.

The term "process parameter" as used herein is a broad term and is to be given its ordinary and customary meaning to a person of ordinary skill in the art and is not to be limited to a special or customized meaning. The term specifically may refer, without limitation, to an arbitrary process related variable characterizing the process. As an example, further process parameters of the compounding process may be regulated, such as compounding parameters adjustable via at least one of the settings of the extruder, e.g. via one or more of a conveying velocity, a rotational speed, such as a rotational speed of the at least one screw, an energy input, for example controlled via a temperature of at least one element of the extruder, such as a temperature of the at least one screw and/or a temperature of the housing.

The method steps b) to d) may specifically be performed by a computer. As generally used herein, the term "computer" may refer to a device having at least one processor and optionally further elements, such as, for example, one or more interfaces, one or more data storage devices, one or more display devices and the like. The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

Further disclosed and proposed herein is a computer program, specifically an application, for controlling polymer viscosity quality in a compounding process of polymers wherein in the compounding process at least one extruder is used. The computer program comprises instructions, which, when the program is executed by a computer or computer network, cause the computer or computer network to carry out the following steps:
- retrieving at least one influence variable affecting viscosity of the compound;
- predicting an expected viscosity of the compound considering the influence variable by using at least one analysis tool comprising at least one trained model;
- comparing the expected viscosity of the compound to at least one pre-defined and/or pre-determined threshold value, wherein at least one item of output information is generated depending on said comparison; and
- displaying the item of output information, wherein the output information comprises at least one handling recommendation for at least one setting of the extruder.

The term "computer network" may generally refer to a system of interconnected electrical devices, wherein at least one of the devices may specifically be a computer.

In a further aspect of the invention, a controlling system for controlling polymer viscosity quality in a compounding process of polymers, wherein in the compounding process at least one extruder is used, is disclosed. The controlling system comprises at least one sensor configured for determining at least one influence variable affecting viscosity of the compound. The controlling system further comprises at least one prediction unit configured for determining an expected viscosity of the compound considering the influence variable, wherein the prediction unit comprises at least one analysis tool comprising at least one trained model. The controlling system comprises at least one evaluation unit configured for comparing the expected viscosity of the compound to at least one pre-defined and/or pre-determined threshold value, wherein the evaluation unit is configured for generating at least one item of output information depending on said comparison. The controlling system further comprises at least one display device configured for displaying the item of output information. The output information comprises at least one handling recommendation for at least one setting of the extruder.

In particular, the sensor may be or may comprise at least one sensor selected from the group consisting of: a pressure sensor, specifically a melt pressure sensor; an inductive sensor, specifically an inductive slot sensor; a thermocouple.

The controlling system may further comprise a regulator unit configured for regulating at least one process parameter of the compounding process depending on the output information. In particular, depending on the item of output information the at least one process parameter of the compounding process, e.g. the extruder setting, may be regulated by using the regulator unit. Specifically, the process parameter may be or may comprise a rotational speed of the extruder. Thus, as an example, when increasing the rotational speed, the viscosity, such as the polymer viscosity, may be reduced and vice versa.

As an example, the regulator unit, for the purpose of regulating the at least one process parameter of the compounding process, such as for controlling and/or regulating the parameter, e.g. the rotational speed of the extruder, may comprise at least one element, such as a mechanical and/or digital controller, e.g. a rotatable knob, a button, a touch display or the like.

The controlling system is configured for performing the method for controlling at least one quality of a compound in a compounding process as described above or as described in further detail below.

The methods and systems according to the present invention may provide a large number of advantages over known methods and systems for controlling polymer viscosity quality in a compounding process of polymers. Thus, specifically, a method for controlling polymer viscosity quality in a compounding process of polymers using at least one extruder as suggested in the present invention may reduce or even avoid waiting periods and/or idle times and may increase production capacity. In particular, the proposed methods and devices may provide for an foresighted identification of future quality mishaps and/or variation in compound quality and may provide efficient and fast counteraction. Thus, the present methods and devices may allow regulating and/or controlling process parameters such as to prevent loss of quality. The present invention may further improve the safety and quality of products and processes, e.g. of compound products and compounding processes, by real-time controlling of compound quality. Further, the present invention may further provide advantages in terms of the environment compared to state-of-the-art methods and devices for controlling polymer viscosity quality in a compoundinq process of polymers. In particular, the present invention may reduce a production of material loss and waist.

In particular, the present invention may allow for a real-time monitoring and/or control of polymer viscosity quality in a compounding process of polymers. In particular, the present invention may overcome a measurement delay, common in state-of-the-art methods and devices. Further, the present invention may provide a more efficient and more economical monitoring and/or control of polymer viscosity quality in a compounding process of polymers.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1:: shows a flow chart of an embodiment of a method for controlling polymer viscosity quality in a compounding process of polymers using at least one extruder;
- Figure 2:: shows an embodiment of an interface of a computer program for controlling polymer viscosity quality in a compounding process of polymers; and
- Figure 3:: shows an embodiment of a controlling system for controlling polymer viscosity quality in a compounding process of polymers.

### Detailed description of the embodiments

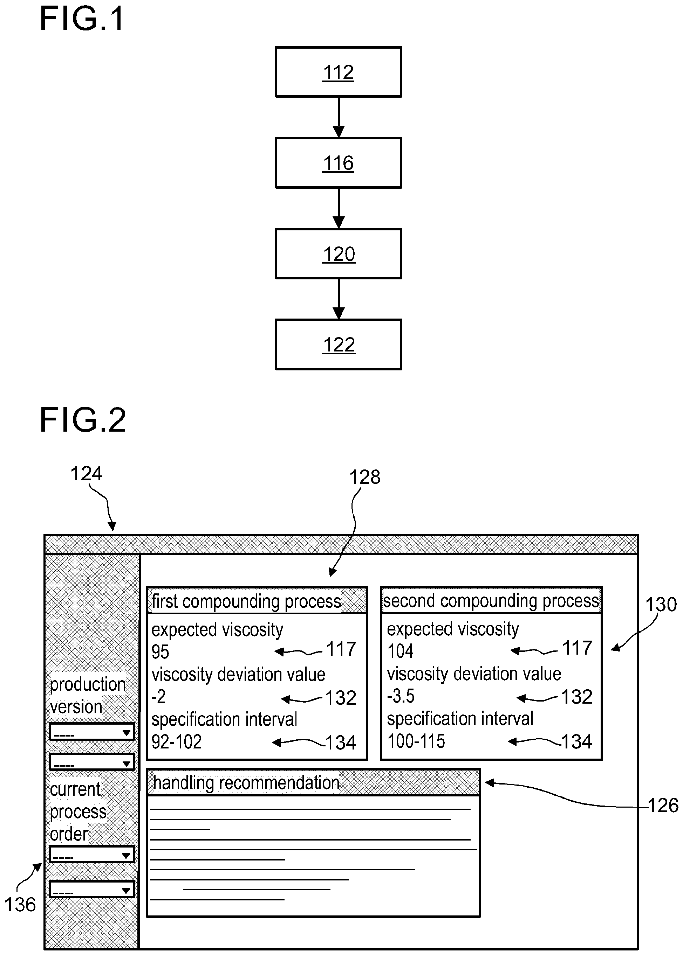

In a first aspect of the invention, a method for controlling polymer viscosity quality in a compounding process of polymers 110 using at least one extruder 111 is disclosed. Figure 1 shows a flow chart of an embodiment of the method. The method comprises the following steps, which may specifically be performed in the given order. Still, a different order may also be possible. It may further be possible to perform two or more of the method steps fully or partially simultaneously. It may further be possible to perform one or more method steps or even all of the method steps once or repeatedly. The method may comprise additional method steps, which are not listed herein. The method steps are the following:
a) at least one measurement step 112, wherein at least one influence variable affecting viscosity of the compound is determined by using at least one sensor 114;
b) at least one prediction step 116, wherein an expected viscosity 117 of the compound is determined considering the influence variable by using at least one prediction unit 118, wherein the prediction unit 118 comprises at least one analysis tool comprising at least one trained model;
c) at least one evaluation step 120, wherein the expected viscosity 117 of the compound is compared to at least one pre-defined and/or pre-determined threshold value, wherein at least one item of output information is generated depending on said comparison; and
d) at least one control step 122, wherein the item of output information is displayed using at least one display device 124, wherein the output information comprises at least one handling recommendation 126 for at least one setting of the extruder 111.

The method may further comprise automatically retrieving information from a database, such as from a sorted accumulation of information, and providing the information to the prediction unit 118. In particular, the retrieving of information from the database may be performed between the measurement step 112 and the prediction step 116.

The measurement step 112 may further comprise at least one pre-processing step, wherein in the pre-processing step, data determined by the sensor 114 may be pre-processed. In particular, the pre-processing may comprise an elimination of outliers. Thus, as an example, outliers may be eliminated from the data determined by the sensor 114 by using at least one clustering algorithm. In particular, the at least one clustering algorithm may be or may comprise one or more of an expectation-maximization algorithm, a k-means algorithm and a k-median algorithm.

The evaluation step 120 may further comprise comparing the expected viscosity 117 of the compound with at least one target specification 121. Therein the target specification may specifically be a customer specification, such as a specification given and/or set by a customer and/or buyer of the compound.

Figure 2 shows an embodiment of an interface of a computer program for controlling polymer viscosity quality in a compounding process of polymers. Specifically, Figure 2 illustrates an interface of a computer or computer network, when an embodiment of a computer program, e.g. an application, for controlling polymer viscosity quality in a compounding process of polymers 110 is executed on the computer or computer network. As an example, the interface may specifically be or may comprise a display device 124, wherein on the display device 124 output information comprising at least one handling recommendation 126 for at least one setting of the extruder 111 may be displayed. As an example, the handling recommendation 126 may be given in text, such as by using letters of an arbitrary language. However, other forms of displaying handling recommendation 126 may be possible, such as by using one or more of a pictogram, a color code, a video-sequence and the like. As an example, the displayed handling recommendation 126 may even be accompanied by at least one audio signal, such as by an acoustic signal, e.g. an acoustic warning signal.

Further, on the interface, e.g. on the display device 124, expected viscosities 117 of more than one compounding process of polymers 110 using at least one extruder 111 may be shown. Thus, as an example, in a first box 128 the expected viscosity 117 of a first compounding process of polymers 110 using at least one extruder 111, e.g. at least one first extruder, may be illustrated, and in a second box 130, the expected viscosity 117 of a second compounding process of polymers 110 using at least one extruder 111, e.g. at least one second extruder, may be illustrated. Further information may be illustrated in the first box 128 and in the second box 130. Such further information may for example be or may comprise one or more of a viscosity deviation value 132 and a specification interval 134. Therein, the specification interval 134 may, for example, be an admissibility range of the viscosity, such as a specification limit and/or boundary. The viscosity deviation value 132, as an example, may specifically be a discrepancy between the expected viscosity 117 and a target specification 121, such as a difference between the expected viscosity 117 and a mean value of the specification interval 134, e.g. a difference between the expected viscosity 117 and a target viscosity value. In a third box 136, at least one input parameter, such as a production version and a current process order, may be illustrated. As an example, based on the input parameters, the computer program may automatically select respective information from a database, such as information on one or more of the trained model, the polymer viscosity quality and a product, e.g. a compound to be produced using the respective compounding process of polymers. Such a selection may be automatically performed, such as in a pre-set interval P. As an example, the pre-set interval P for automatically performing the selection may be 0 min < P ≤ 5 min, specifically 0,5 min ≤ P ≤ 3 min, more specifically 1 min ≤ P ≤ 2 min.

As an example, a color of the first box 128 and the second box 130 may be adapted according to a confidence interval. Thus, as an example, in case a 95%-confidence interval may be within the specification interval 134, the color of the respective box, e.g. of the first box 128 and/or of the second box 130, may comprise a green color. In case the specification interval 134 is violated by at least one limit of the 95%-confidence interval, in particular in case the 95%-confidence interval reaches and/or cuts the specification interval 134, the color of the respective box may be or may comprise a yellow or orange color. Further, in case the 95%-confidence interval may be outside of the specification interval 134, the respective box may comprise a red color.

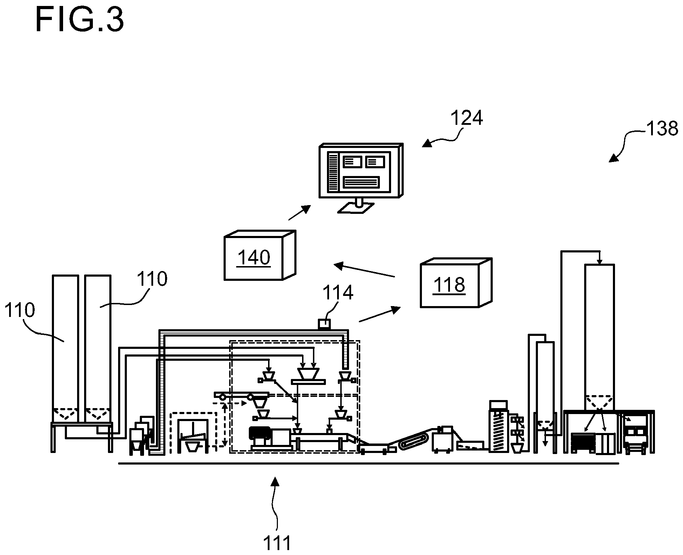

In Figure 3, an embodiment of a controlling system 138 for controlling polymer viscosity quality in a compounding process of polymers 110 is illustrated, wherein in the compounding process at least one extruder 111 is used. The controlling system comprises at least one sensor 114 configured for determining at least one influence variable affecting viscosity of the compound. The controlling system 138 further comprises at least one prediction unit 118 configured for determining an expected viscosity 117 of the compound considering the influence variable, wherein the prediction unit 118 comprises at least one analysis tool comprising at least one trained model. The controlling system 138 further comprises at least one evaluation unit 140 configured for comparing the expected viscosity 117 of the compound to at least one pre-defined and/or pre-determined threshold value, wherein the evaluation unit 140 is configured for generating at least one item of output information depending on said comparison. The controlling system 138 further comprises at least one display device 124 configured for displaying the item of output information. The output information comprises at least one handling recommendation 126 for at least one setting of the extruder 111.

The controlling system 138 may further comprise a regulator unit (not illustrated in Figure 3) configured for regulating at least one process parameter of the compounding process depending on the output information. In particular, depending on the item of output information the at least one process parameter of the compounding process, e.g. the extruder setting, may be regulated by using the regulator unit. Specifically, the process parameter may be or may comprise a rotational speed of the extruder 111. Thus, as an example, when increasing the rotational speed, the viscosity, such as the polymer viscosity, may be reduced and vice versa.

The controlling system 138 may be configured for performing the method for controlling at least one quality of a compound in a compounding process as illustrated in Figure 1.

### List of reference numbers

- 110: polymer
- 111: extruder
- 112: measurement step
- 114: sensor
- 116: prediction step
- 117: expected viscosity
- 118: prediction unit
- 120: evaluation step
- 121: target specification
- 122: control step
- 124: display device
- 126: handling recommendation
- 128: first box
- 130: second box
- 132: viscosity deviation value
- 134: specification interval
- 136: third box
- 138: controlling system
- 140: evaluation unit

### List of cited references

- "A novel approach to dynamic modelling of polymer extrusion for improved process control" on pages 617 - 628 in "proceedings of the Institution of Mechanical Engineers, Vol. 221 Part I: J. Systems and Control Engineering", by McAfee and Thompson
- "Soft-sensor for real-time monitoring of melt viscosity in polymer extrusion process" in "49th IEEE Conference on decision and Control", by Liu et al.
- "Bestandigkeit von Kunststoffen", Munich: Hanser 2007, by Gottfried W. Ehrenstein and Sonja Pongratz
- Computers & Chemical Engineering, 60, Pages 86 to 101, 2014, by Von Stoch et al.
- "Random Forests" in Machine Learning 45.1, Oct. 2001, by Leo Breiman
- "The Elements of Statistical Learning", Springer New York, 2009, by Trevor Hastie, Robert Tibshirani and Jerome Friedman
- "Conditional variable importance for random forests" in BMC Bioinformatics 9.1, July 2008, page 307, by Carolin Strobl et al;
   - WO 2008/040943 A2;
   - ES2 331 720 A1;
   - EP 3 020 530 A1;
- Kumar A. et al.: "A model based approach for estimation and control for polymer compounding", Proceedings of the 2003 IEEE International Conference on Control Applications, CCA 2003, ISTANBUL, TURKEY, JUNE 23 - 25, 2003; NEW YORK, NY: IEEE, US, vol. I, 23 June 2003, pages 729-735, XP010652701, ISBN: 978-0-7803-7729-5.

## Claims

1. A method for controlling polymer viscosity quality in a compounding process of polymers (110) using at least one extruder (111), wherein the compound at least partially comprises at least one thermoplastic material selected from the group consisting of: polybutylene terephthalate (PBT); polyethylene terephthalate (PET); polyamide (PA); polystyrene (PS), the method comprising:
a) at least one measurement step (112), wherein at least one influence variable affecting viscosity of the compound is determined by using at least one sensor (114);
b) at least one prediction step (116), wherein an expected viscosity (117) of the compound is determined considering the influence variable by using at least one prediction unit (118), wherein the prediction unit (118) comprises at least one analysis tool comprising at least one trained model;
c) at least one evaluation step (120), wherein the expected viscosity (117) of the compound is compared to at least one pre-defined and/or pre-determined threshold value, wherein at least one item of output information is generated depending on said comparison; and
d) at least one control step (122), wherein the item of output information is displayed using at least one display device (124), wherein the output information comprises at least one handling recommendation (126) for at least one setting of the extruder (111);
**characterised in that**:
i) if the at least one expected viscosity (117) of the compound falls within a predefined tolerance range of the threshold value, the handling recommendation (126) comprises information on maintaining the setting of the extruder (111); and
ii) if the at least one expected viscosity (117) of the compound differs from the threshold value by more than the predefined tolerance range, the handling recommendation (126) comprises information on changing the setting of the extruder (111),
wherein the predefined tolerance range is one or both of a relative and an absolute tolerance, wherein the predefined tolerance range is ± 5 %, preferably ± 2 %, and/or ± 3 ml/g, preferably ± 2 ml/g.

2. The method according to the preceding claim, wherein the influence variable is selected from the group consisting of: a head pressure; a torque; a temperature; a transfer rate; a rotational speed; a shear rate; a mass flow; an electric power consumption.

3. The method according to any one of the preceding claims, wherein the method comprises generating at least one database, wherein the database comprises one or both of quantitative and qualitative information on the compounding process, wherein the information is selected from the group consisting of: sensor data; a material; a process parameter of the compounding process; a pre-determined target viscosity value.

4. The method according to any one of the preceding claims, wherein the trained model comprises at least one model selected from the group consisting of: at least one random forest algorithm; at least one neural network; at least one linear model; at least one non-linear model; at least one grey box model; at least one elastic net algorithm.

5. The method according to any one of the preceding claims, wherein step a) further comprises at least one pre-processing step, wherein data determined by the sensor (114) is pre-processed, wherein the pre-processing comprises eliminating of outliers, wherein outliers are eliminated from the data determined by the sensor (114) by using at least one clustering algorithm.

6. The method according to any one of the preceding claims, wherein the output information further comprises at least one information selected from the group consisting of: an information on the expected viscosity of the compound; an information on historical viscosity of the compound such as measured viscosity and/or expected viscosity.

7. The method according to any one of the preceding claims, wherein the method further comprises regulating at least one process parameter of the compounding process depending on the output information, wherein the process parameter comprises at least one rotational speed of the extruder (111).

8. A computer program for controlling polymer viscosity quality in a compounding process of polymers (110), wherein in the compounding process at least one extruder (111) is used, wherein the compound at least partially comprises at least one thermoplastic material selected from the group consisting of: polybutylene terephthalate (PBT); polyethylene terephthalate (PET); polyamide (PA); polystyrene (PS), the computer program comprises instructions which, when the program is executed by a computer or computer network, cause the computer or computer network to carry out the following steps
- retrieving at least one influence variable affecting viscosity of the compound;
- predicting an expected viscosity (117) of the compound considering the influence variable by using at least one analysis tool comprising at least one trained model;
- comparing the expected viscosity (117) of the compound to at least one pre-defined and/or pre-determined threshold value, wherein at least one item of output information is generated depending on said comparison; and
- displaying the item of output information, wherein the output information comprises at least one handling recommendation (126) for at least one setting of the extruder (111), wherein, if the at least one expected viscosity (117) of the compound falls within a predefined tolerance range of the threshold value, the handling recommendation (126) comprises information on maintaining the setting of the extruder (111), and **characterised in that**, if the at least one expected viscosity (117) of the compound differs from the threshold value by more than the predefined tolerance range, the handling recommendation (126) comprises information on changing the setting of the extruder (111),wherein the predefined tolerance range is one or both of a relative and an absolute tolerance, wherein the predefined tolerance range is ± 5 %, preferably ± 2 %, and/or ± 3 ml/g, preferably ± 2 ml/g.

9. A controlling system (138) for controlling polymer viscosity quality in a compounding process of polymers (110), wherein the controlling system (138) is configured for performing the method for controlling at least one quality of a compound in a compounding process according to any one of the preceding claims referring to a method, wherein the compound at least partially comprises at least one thermoplastic material selected from the group consisting of: polybutylene terephthalate (PBT); polyethylene terephthalate (PET); polyamide (PA); polystyrene (PS), wherein in the compounding process at least one extruder (111) is used, wherein the controlling system (138) comprises at least one sensor (114) configured for determining at least one influence variable affecting viscosity of the compound, wherein the controlling system (138) comprises at least one prediction unit (118) configured for determining an expected viscosity (117) of the compound considering the influence variable, wherein the prediction unit (118) comprises at least one analysis tool comprising at least one trained model, wherein the controlling system (138) comprises at least one evaluation unit (140) configured for comparing the expected viscosity (117) of the compound to at least one pre-defined and/or pre-determined threshold value, wherein the evaluation unit (140) is configured for generating at least one item of output information depending on said comparison, wherein the controlling system (138) comprises at least one display device (124) configured for displaying the item of output information, wherein the output information comprises at least one handling recommendation (126) for at least one setting of the extruder (111).

10. The controlling system (138) according to the preceding claim, wherein the sensor (114) comprises at least one sensor (114) selected from the group consisting of: a pressure sensor, specifically a melt pressure sensor; an inductive sensor, specifically an inductive slot sensor; a thermocouple.

11. The controlling system (138) according to any one of the two preceding claims, wherein the controlling system (138) further comprises a regulator unit configured for regulating at least one process parameter of the compounding process depending on the output information, wherein the process parameter comprises at least one rotational speed of the extruder (111).

## Patentansprüche

1. Verfahren zum Steuern der Polymerviskositätseigenschaft bei einem Mischverfahren von Polymeren (110) unter Verwendung wenigstens eines Extruders (111), wobei die Mischung wenigstens zum Teil wenigstens ein thermoplastisches Material ausgewählt aus der Gruppe bestehend aus: Polybutylenterephthalat (PBT); Polyethylenterephthalat (PET); Polyamid (PA); Polystyrol (PS) umfasst, wobei das Verfahren umfasst:
a) wenigstens einen Messschritt (112), wobei wenigstens eine Einflussvariable, die die Viskosität der Mischung beeinflusst, unter Verwendung wenigstens eines Sensors (114) bestimmt wird;
b) wenigstens einen Vorhersageschritt (116), wobei eine erwartete Viskosität (117) der Mischung unter Berücksichtigung der Einflussvariable unter Verwendung wenigstens einer Vorhersageeinheit (118) bestimmt wird, wobei die Vorhersageeinheit (118) wenigstens ein Analysewerkzeug umfasst, das wenigstens ein trainiertes Modell umfasst;
c) wenigstens einen Auswertungsschritt (120), wobei die erwartete Viskosität (117) der Mischung mit wenigstens einem vorgegebenen und/oder vorbestimmten Schwellenwert verglichen wird, wobei wenigstens ein Ausgabeinformationselement abhängig von dem Vergleich erzeugt wird; und
d) wenigstens einen Steuerschritt (122), wobei das Ausgabeinformationselement unter Verwendung wenigstens einer Anzeigevorrichtung (124) angezeigt wird, wobei die Ausgabeinformation wenigstens eine Bedienungsempfehlung (126) für wenigstens eine Einstellung des Extruders (111) umfasst;
**dadurch gekennzeichnet, dass**:
i) wenn die wenigstens eine erwartete Viskosität (117) der Mischung in einen vorgegebenen Toleranzbereich der Schwellenwerts fällt, die Bedienungsempfehlung (126) Informationen zum Beibehalten der Einstellung des Extruders (111) umfasst; und
ii) wenn die wenigstens eine erwartete Viskosität (117) der Mischung von dem vorgegebenen Schwellenwert um mehr als den vorgegebenen Toleranzbereich abweicht, die Bedienungsempfehlung (126) Informationen zum Verändern der Einstellung des Extruders (111) umfasst;
wobei der vorgegebene Toleranzbereich eines oder beide von einer relativen und einer absoluten Toleranz ist, wobei der vorgegebene Toleranzbereich ± 5 %, vorzugsweise ± 2 %, und/oder ± 3 ml/g, vorzugsweise ± 2 mg/l, beträgt.

2. Verfahren gemäß dem vorstehenden Anspruch, wobei die Einflussvariable ausgewählt ist aus der Gruppe bestehend aus: einem Kopfdruck; einem Drehmoment; einer Temperatur; einer Überführungsrate; einer Drehgeschwindigkeit; einer Scherrate; einem Massefluss; einer elektrischen Leistungsaufnahme.

3. Verfahren gemäß einem der vorstehenden Ansprüche, wobei das Verfahren Erzeugen wenigstens einer Datenbank umfasst, wobei die Datenbank eines oder beide von quantitativen und qualitativen Informationen über das Mischverfahren umfasst, wobei die Informationen ausgewählt sind aus der Gruppe bestehend aus: Sensordaten; einem Material; einem Prozessparameter des Mischverfahrens; einem vorgegebenen Ziel-Viskositätswert.

4. Verfahren gemäß einem der vorstehenden Ansprüche, wobei das trainierte Modell wenigstens ein Modell ausgewählt aus der Gruppe bestehend aus: wenigstens einem Random-Forest-Algorithmus; wenigstens einem neuronalen Netz; wenigstens einem linearen Modell; wenigstens einem nichtlinearen Modell; wenigstens einem Grey-Box-Modell; wenigstens einem Elastic-Net-Algorithmus umfasst.

5. Verfahren gemäß einem der vorstehenden Ansprüche, wobei Schritt a) ferner wenigstens einen Vorverarbeitungsschritt umfasst, wobei die von dem Sensor (114) bestimmten Daten vorverarbeitet werden, wobei die Vorverarbeitung Eliminieren von Ausreißern umfasst, wobei Ausreißer unter Verwendung wenigstens eines Clustering-Algorithmus aus den von dem Sensor (114) bestimmten Daten eliminiert werden.

6. Verfahren gemäß einem der vorstehenden Ansprüche, wobei die Ausgabeinformationen ferner wenigstens eine Information ausgewählt aus der Gruppe bestehend aus: einer Information über die erwartete Viskosität der Mischung; einer Information über historische Viskosität der Mischung, wie z.B. gemessene Viskosität und/oder erwartete Viskosität, umfasst.

7. Verfahren gemäß einem der vorstehenden Ansprüche, wobei das Verfahren ferner Regulieren wenigstens eines Prozessparameters des Mischverfahrens abhängig von den Ausgabeinformationen umfasst, wobei der Prozessparameter wenigstens eine Drehgeschwindigkeit des Extruders (111) umfasst.

8. Computerprogramm zum Steuern der Polymerviskositätseigenschaft bei einem Mischverfahren von Polymeren (110), wobei bei dem Mischverfahren wenigstens ein Extruder (111) verwendet wird, wobei die Mischung wenigstens zum Teil wenigstens ein thermoplastisches Material ausgewählt aus der Gruppe bestehend aus: Polybutylenterephthalat (PBT); Polyethylenterephthalat (PET); Polyamid (PA); Polystyrol (PS) umfasst, wobei das Computerprogramm Anweisungen umfasst, die bei Ausführung des Programms durch einen Computer oder ein Computernetzwerk bewirkt, dass der Computer oder das Computernetzwerk die folgenden Schritte ausführt:
- Erfassen wenigstens einer Einflussvariable, die die Viskosität der Mischung beeinflusst,
- Vorhersagen einer erwarteten Viskosität (117) der Mischung unter Berücksichtigung der Einflussvariable unter Verwendung wenigstens eines Analysewerkzeugs, das wenigstens ein trainiertes Modell umfasst;
- Vergleichen der erwarteten Viskosität (117) der Mischung mit wenigstens einem vorgegebenen und/oder vorbestimmten Schwellenwert, wobei wenigstens ein Ausgabeinformationselement abhängig von dem Vergleich erzeugt wird; und
- Anzeigen des Ausgabeinformationselements, wobei die Ausgabeinformation wenigstens eine Bedienungsempfehlung (126) für wenigstens eine Einstellung des Extruders (111) umfasst;
wobei, wenn die wenigstens eine erwartete Viskosität (117) der Mischung in einen vorgegebenen Toleranzbereich der Schwellenwerts fällt, die Bedienungsempfehlung (126) Informationen zum Beibehalten der Einstellung des Extruders (111) umfasst, und **dadurch gekennzeichnet, dass**, wenn die wenigstens eine erwartete Viskosität (117) der Mischung von dem Schwellenwert um mehr als den vorgegebenen Toleranzbereich abweicht, die Bedienungsempfehlung (126) Informationen zum Verändern der Einstellung des Extruders (111) umfasst, wobei der vorgegebene Toleranzbereich eines oder beide von einer relativen und einer absoluten Toleranz ist, wobei der vorgegebene Toleranzbereich ± 5 %, vorzugsweise ± 2 %, und/oder ± 3 ml/g, vorzugsweise ± 2 mg/l, beträgt.

9. Steuersystem (138) zum Steuern der Polymerviskositätseigenschaft bei einem Mischverfahren von Polymeren (110), wobei das Steuersystem (138) dafür gestaltet ist, das Verfahren zum Steuern wenigstens einer Eigenschaft einer Mischung bei einem Mischverfahren gemäß wenigstens einem der vorstehenden Ansprüche, die sich auf ein Verfahren beziehen, durchzuführen, wobei die Mischung wenigstens zum Teil wenigstens ein thermoplastisches Material ausgewählt aus der Gruppe bestehend aus: Polybutylenterephthalat (PBT); Polyethylenterephthalat (PET); Polyamid (PA); Polystyrol (PS) umfasst, wobei bei dem Mischverfahren wenigstens ein Extruder (111) verwendet wird, wobei das Steuersystem (138) wenigstens einen Sensor (114) umfasst, der dafür gestaltet ist, wenigstens eine Einflussvariable zu bestimmen, die die Viskosität der Mischung beeinflusst, wobei das Steuersystem (138) wenigstens eine Vorhersageeinheit (118) umfasst, die dafür gestaltet ist, eine erwartete Viskosität (117) der Mischung unter Berücksichtigung der Einflussvariable zu bestimmen, wobei die Vorhersageeinheit (118) wenigstens ein Analysewerkzeug umfasst, das wenigstens ein trainiertes Modell umfasst, wobei das Steuersystem (138) wenigstens eine Auswertungseinheit (140) umfasst, die dafür gestaltet ist, die erwartete Viskosität (117) der Mischung mit wenigstens einem vorgegebenen und/oder vorbestimmten Schwellenwert zu vergleichen, wobei die Auswertungseinheit (140) dafür gestaltet ist, wenigstens ein Ausgabeinformationselement abhängig von dem Vergleich zu erzeugen, wobei das Steuersystem (138) wenigstens eine Anzeigevorrichtung (124) umfasst, die dafür gestaltet ist, das Ausgabeinformationselement anzuzeigen, wobei die Ausgabeinformation wenigstens eine Bedienungsempfehlung (126) für wenigstens eine Einstellung des Extruders (111) umfasst.

10. Steuersystem (138) gemäß dem vorstehenden Anspruch, wobei der Sensor (114) wenigstens einen Sensor (114) ausgewählt aus der Gruppe bestehend aus: einem Drucksensor, insbesondere einem Schmelzedrucksensor; einem induktiven Sensor, insbesondere einem induktiven Schlitzsensor, einem Thermoelement umfasst.

11. Steuersystem (138) gemäß einem der beiden vorstehenden Ansprüche, wobei das Steuersystem (138) ferner eine Reglereinheit umfasst, die dafür gestaltet ist, wenigstens einen Prozessparameter des Mischverfahrens abhängig von der Ausgabeinformation zu regulieren, wobei der Prozessparameter wenigstens eine Drehgeschwindigkeit des Extruders (111) umfasst.

## Revendications

1. Procédé permettant de commander la qualité de la viscosité d'un polymère dans un processus de compoundage de polymères (110) en utilisant au moins une extrudeuse (111), dans lequel le composé comprend au moins partiellement au moins un matériau thermoplastique choisi dans le groupe constitué par : le polybutylène téréphtalate (PBT) ; le polyéthylène téréphtalate (PET) ; le polyamide (PA) ; le polystyrène (PS), le procédé comprenant :
a) au moins une étape de mesure (112), dans laquelle au moins une variable d'influence affectant la viscosité du composé est déterminée en utilisant au moins un capteur (114) ;
b) au moins une étape de prédiction (116), dans laquelle une viscosité attendue (117) du composé est déterminée en tenant compte de la variable d'influence en utilisant au moins une unité de prédiction (118), l'unité de prédiction (118) comprenant au moins un outil d'analyse comprenant au moins un modèle entraîné ;
c) au moins une étape d'évaluation (120), dans laquelle la viscosité attendue (117) du composé est comparée à au moins une valeur seuil prédéfinie et/ou prédéterminée, dans laquelle au moins un élément d'informations de sortie est généré en fonction de ladite comparaison ; et
d) au moins une étape de commande (122), dans laquelle l'élément d'informations de sortie est affiché en utilisant au moins un dispositif d'affichage (124), dans laquelle les informations de sortie comprennent au moins une recommandation de manipulation (126) pour au moins un réglage de l'extrudeuse (111) ;
**caractérisé en ce que** :
i) si l'au moins une viscosité attendue (117) du composé s'inscrit dans une plage de tolérance prédéfinie de la valeur seuil, la recommandation de manipulation (126) comprend des informations sur le maintien du réglage de l'extrudeuse (111) ; et
ii) si l'au moins une viscosité attendue (117) du composé diffère de la valeur seuil de plus de la plage de tolérance prédéfinie, la recommandation de manipulation (126) comprend des informations sur le changement du réglage de l'extrudeuse (111),
dans lequel la plage de tolérance prédéfinie est une tolérance relative et/ou une tolérance absolue, dans lequel la plage de tolérance prédéfinie est de ±5 %, de préférence ±2 %, et/ou ±3 ml/g, de préférence ±2 ml/g.

2. Procédé selon la revendication précédente, dans lequel la variable d'influence est choisie dans le groupe constitué par : une pression de tête ; un couple ; une température ; un taux de transfert ; une vitesse de rotation ; un taux de cisaillement ; un débit massique ; une consommation électrique.

3. Procédé selon l'une quelconque des revendications précédentes, le procédé comprenant la génération d'au moins une base de données, dans lequel la base de données comprend des informations quantitatives et/ou qualitatives sur le processus de compoundage, dans lequel les informations sont choisies dans le groupe constitué par : des données de capteur ; un matériau ; un paramètre de processus du processus de compoundage ; une valeur de viscosité cible prédéterminée.

4. Procédé selon l'une quelconque des revendications précédentes, dans lequel le modèle entraîné comprend au moins un modèle choisi dans le groupe constitué par : au moins un algorithme de forêt aléatoire ; au moins un réseau neuronal ; au moins un modèle linéaire ; au moins un modèle non linéaire ; au moins un modèle de boîte grise ; au moins un algorithme de filet élastique.

5. Procédé selon l'une quelconque des revendications précédentes, dans lequel l'étape a) comprend en outre au moins une étape de prétraitement, dans laquelle les données déterminées par le capteur (114) sont prétraitées, dans laquelle le prétraitement comprend l'élimination de valeurs aberrantes, dans laquelle les valeurs aberrantes sont éliminées des données déterminées par le capteur (114) en utilisant au moins un algorithme de groupement.

6. Procédé selon l'une quelconque des revendications précédentes, dans lequel les informations de sortie comprennent en outre au moins des informations choisies dans le groupe constitué par : des informations sur la viscosité attendue du composé ; des informations sur la viscosité historique du composé telle que la viscosité mesurée et/ou la viscosité attendue.

7. Procédé selon l'une quelconque des revendications précédentes, le procédé comprenant en outre la régulation d'au moins un paramètre de processus du processus de compoundage en fonction des informations de sortie, le procédé comprenant en outre au moins une vitesse de rotation de l'extrudeuse (111).

8. Programme d'ordinateur permettant de commander la qualité de la viscosité d'un polymère dans un processus de compoundage de polymères (110), dans lequel, dans le processus de compoundage, au moins une extrudeuse (111) est utilisée, dans lequel le composé comprend au moins partiellement au moins un matériau thermoplastique choisi dans le groupe constitué par : le polybutylène téréphtalate (PBT) ; le polyéthylène téréphtalate (PET) ; le polyamide (PA) ; le polystyrène (PS), le programme d'ordinateur comprend des instructions qui, lorsque le programme est exécuté par un ordinateur ou un réseau d'ordinateurs, amènent l'ordinateur ou le réseau d'ordinateurs à effectuer les étapes suivantes
- la récupération d'au moins une variable d'influence affectant la viscosité du composé ;
- la prédiction d'une viscosité attendue (117) du composé en tenant compte de la variable d'influence en utilisant au moins un outil d'analyse comprenant au moins un modèle entraîné ;
- la comparaison de la viscosité attendue (117) du composé à au moins une valeur seuil prédéfinie et/ou prédéterminée, l'au moins un élément d'informations de sortie étant généré en fonction de ladite comparaison ; et
- l'affichage de l'élément d'informations de sortie, les informations de sortie comprenant au moins une recommandation de manipulation (126) pour au moins un réglage de l'extrudeuse (111), dans lequel, si l'au moins une viscosité attendue (117) du composé s'inscrit dans une plage de tolérance prédéfinie de la valeur seuil, la recommandation de manipulation (126) comprend des informations sur le maintien du réglage de l'extrudeuse (111), et **caractérisé en ce que**, si l'au moins une viscosité attendue (117) du composé diffère de la valeur seuil de plus de la plage de tolérance prédéfinie, la recommandation de manipulation (126) comprend des informations sur le changement du réglage de l'extrudeuse (111), dans lequel la plage de tolérance prédéfinie est une tolérance relative et/ou une tolérance absolue, dans lequel la plage de tolérance prédéfinie est de ±5 %, de préférence ±2 %, et/ou de ±3 ml/g, de préférence ±2 ml/g.

9. Système de commande (138) permettant de commander la qualité de la viscosité d'un polymère dans un processus de compoundage de polymères (110), dans lequel le système de commande (138) est configuré pour réaliser le procédé de commande d'au moins une qualité d'un composé dans un processus de compoundage selon l'une quelconque des revendications précédentes se référant à un procédé, dans lequel le composé comprend au moins partiellement un matériau thermoplastique sélectionné dans le groupe constitué par : le polybutylène téréphtalate (PBT) ; le polyéthylène téréphtalate (PET) ; le polyamide (PA) ; le polystyrène (PS), dans lequel, dans le processus de compoundage, au moins une extrudeuse (111) est utilisée, dans lequel le système de commande (138) comprend au moins un capteur (114) configuré pour déterminer au moins une variable d'influence affectant la viscosité du composé, dans lequel le système de commande (138) comprend au moins une unité de prédiction (118) configurée pour déterminer une viscosité attendue (117) du composé en tenant compte de la variable d'influence, dans lequel l'unité de prédiction (118) comprend au moins un outil d'analyse comprenant au moins un modèle entraîné, dans lequel le système de commande (138) comprend au moins une unité d'évaluation (140) configurée pour comparer la viscosité attendue (117) du composé à au moins une valeur seuil prédéfinie et/ou prédéterminée, dans lequel l'unité d'évaluation (140) est configurée pour générer au moins un élément d'informations de sortie en fonction de ladite comparaison, dans lequel le système de commande (138) comprend au moins un dispositif d'affichage (124) configuré pour afficher l'élément d'informations de sortie, dans lequel les informations de sortie comprennent au moins une recommandation de manipulation (126) pour au moins un réglage de l'extrudeuse (111).

10. Système de commande (138) selon la revendication précédente, dans lequel le capteur (114) comprend au moins un capteur (114) choisi dans le groupe constitué par : un capteur de pression, plus particulièrement un capteur de pression de fusion ; un capteur inductif, plus particulièrement un capteur inductif à fente ; un thermocouple.

11. Système de commande (138) selon l'une quelconque des deux revendications précédentes, le système de commande (138) comprenant en outre une unité de régulateur configurée pour réguler au moins un paramètre de processus du processus de compoundage en fonction des informations de sortie, dans lequel le paramètre de processus comprend au moins une vitesse de rotation de l'extrudeuse (111).
